# EUROPEAN PATENT SPECIFICATION

(11) **EP 1 015 568 B1**
(45) Date of publication and mention of the grant of the patent: **26.02.2003**
(21) Application number: 97901624.3
(22) Date of filing: 31.01.1997
(51) Int. Cl.: C12N 9/72, C07K 1/16, A61K 38/49

(54) **METHOD FOR THE PRODUCTION OF rDSPA Alpha1**
VERFAHREN ZUR HERSTELLUNG VON rDSPA Alpha1
PROCEDE DE PRODUCTION DE rDSPA Alpha1

(30) Priority: 05.02.1996 US 597059
(43) Date of publication of application: 05.07.2000
(73) Proprietor: SCHERING AKTIENGESELLSCHAFT, 13353 Berlin (DE)
(72) Inventor: MCCAMAN, Michael, San Bruno, CA 94066 (US); PUNGOR, Erno, Millbrae, CA 94030 (US); SOUDERS, Carol, Los Altos, CA 94022 (US); TAN, Mei, P., San Mateo, CA 94404 (US)
(86) International application number: PCT/EP97/00441
(87) International publication number: WO 97/029188

(56) References cited:
- EP-A- 0 352 119
- BLOOD, vol. 79, no. 5, 1 March 1992, pages 1213-1217, XP000671388 WERNER WITT ET AL.: "Thrombolytic properties of Desmodus rotundus (vampire bat) salivary plasminogen activator in experimantal pulmonary embolism in rats" cited in the application
- J. BIOTECHNOL. (1995), 39(1), 75-83 CODEN: JBITD4;ISSN: 0168-1656, 1995, XP002030441 PETRI, T. ET AL: "Production of vampire bat plasminogen activator DSPA.alpha.1 in CHO and insect cells"
- ANN. N. Y. ACAD. SCI. (1992), 667(PLASMINOGEN ACTIVATION IN FIBRINOLYSIS, IN TISSUE REMODELING, AND IN DEVELOPMENT), 395-403 CODEN: ANYAA9;ISSN: 0077-8923, 1992, XP000671393 SCHLEUNING, WOLF DIETER ET AL: "Plasminogen activators from the saliva of Desmodus rotundus (common vampire bat): unique fibrin specificity"
- METHODS ENZYMOL. (1993), 223(PROTEOLYTIC ENZYMES IN COAGULATION, FIBRINOLYSIS, AND COMPLEMENT ACTIVATION, PT. B.), 233-49 CODEN: MENZAU;ISSN: 0076-6879, 1993, XP000671394 GARDELL, STEPHEN J. ET AL: "Vampire bat salivary plasminogen activator"

## Description

### Field of the Invention

The present invention is directed to a method for the isolation and purification of recombinant *Desmondus rotundus* salivary plasminogen activator α1 (rDSPA α1).

### BACKGROUND OF THE INVENTION

Thromboses are produced by the formation of a blood clot in blood vessels. One distinguishes between venous thromboses induding pulmonary embolisms and arterial thromboses including acute myocardial infarction. Pulmonary embolism and cardiac infarction are life-threatening events requiring immediate medical intervention.

A popular form of therapy for such arterial and venous thromboses is the use of plasminogen activators to perform enzymatic thrombolysis (Collen et al., *Ann. Rev. Med*., (1988), 39: 405-423). These substances, called thrombolytics, convert plasminogen, the inactive proenzyme of the fibrinolysis system in the blood, into the active proteolytic enzyme, plasmin. Plasmin, in turn, dissolves the fibrous substance fibrin, which is a substantial component of a blood clot; this leads to reopening of the blocked vessels and restoration of blood flow. However, because plasmin is a relatively nonspecific protease, it can also destroy, through proteolysis, components in the blood indispensable for intact hemostasis (e.g. fibrinogen) and thus increase the risks of hemorrhaging.

The first enzymatic thrombolytics, streptokinase and urokinase, are compounds which, once injected into the circulation, systemically convert plasminogen into plasmin and induce systemic proteolysis. Thus, thrombolysis therapies which use these compounds are accompanied by the complications related to hemorrhage. Newer thrombolytic therapies, based on the use of plasminogen activators of the tissue type, commonly referred to as t-PA, have been developed but they are also beset by a number of drawbacks, induding serious bleeding complications, a relatively frequent incidence of reocdusion, an inability to be uniformly effective, and susceptibility to inactivation by plasminogen activator inhibitors such as Type 1 plasminogen activator inhibitor (PA1-1) (Loskutoff, *Seminars in Thrombosis and Hemostasis,* Vol. 14, No. 1 (1988)).

More recently, plasminogen activator proteins have been purified from vampire bat (*Desmondus rotundus*) saliva and salivary glands (European Published Patent Application 0 383 417 (Baldus et al.); European Published Patent Application 0 352 119 (Duong et al.)). These plasminogen activators (referred to as DSPA) are serine proteases which catalyze the conversion of plasminogen to plasmin but they exhibit greater selectivity towards fibrin-bound plasminogen and, hence, may be associated with decreased severity and frequency of bleeding when used for thombolytic therapy. Furthermore, DSPA is not readily inactivated by plasma inhibitors such as PAI-1, and therefore, may be associated with a lower frequency of reocclusion.
Two high molecular weight forms of DSPA (designated α1 and α2) can be found in bat saliva, both of which consist of several domains, including a protease domain, and both of which are capable of tightly binding to plasminogen in the presence of fibrin. The various forms of DSPA have been produced in mammalian cell culture by recombinant biotechnology (Kratzschmer et al., Gene 1991), 105:229-237; European Published Patent Application 0 352 119 (Duong et al.)) and small scale purification of recombinantly produced DSPA (rDSPA) has been described (Witt et al., Blood (1992), 79: 1213-1217). However, the isolation and purification of rDSPA on a commercial scale and in a state of purity suitable for pharmaceutical formulations has not been disclosed.
Petri, T. et al. (J. Biotechnology (1995), 39(1), 75-83 describe a method for purifying recombinant DSPAα1 by affinity chromatography using Erythrina latissima trypsin inhibitor immobilized to Sepharose. However, said authors do not describe the elimination of potential viral contaminants, which is necessary for a composition suitable for clinical use.
The instant application is concerned with isolation and purification of recombinant DSPAα1 (rDSPAα1) on a commercial scale. The invention as described results in rDSPAα1 sufficiently pure and stable to be sold commercially and to be clinically usable.

### SUMMERY OF THE INVENTION

The present invention provides a method for the isolation and purification of rDSPAα1 on a commercial scale, and results in product which is suitable for clinical use.

Accordingly, an aspect of the invention is directed to a method for purifying recombinant Desmodus rotundus salivary plasminogen activator (rDSPAα1) from a biological medium, the method comprising the following steps:
(a) applying the medium to a cation exchange resin at a pH between 4 and 7;
(b) washing the cation exchange resin to remove non-rDSPAα1 proteins and non-protein contaminants;
(c) selectively eluting the bound rDSPAα1 from the cation exchange resin;
(d) applying the rDSPAα1-containing eluent from step (c) to a hydrophobic interaction resin at a pH between 3 and 5;
(e) washing the hydrophobic interaction resin to remove non-rDSPAα1 protein and non-protein contaminants;
(f) selectively eluting the bound rDSPAα1 from the hydrophobic interaction resin;
(g) appyling the rDSPAα1-containing eluent from step (f) to an affinity chromatography resin at low pH and low ionic strength;
(h) washing the affinity chromatography resin to remove non-rDSPAα1 protein and non-protein contaminants;
(i) selectively eluting the bound rDSPAα1 from the affinity chromatography resin to produce pure rDSPAα1 in an aqueous solution.

### DETAILED DESCRIPTION OF THE INVENTION

As used in the specification and appended claims, unless specified to the contrary, the following terms have the meaning indicated:

"Biological medium" refers to a precise recipe of salts and nutrients used to propagate cells in culture.

"Conditioned medium" refers to a biological medium in which cells have been grown. The medium has, therefore, been conditioned by the growth of the cells and contains products excreted into the medium during cell growth. These can be both waste products produced during growth or proteins which have been made and secreted into the medium by the cells during growth.

"Cation exchange resin" refers to a natural or artificial substance, usually a solid, which is able to exchange bound ions for ions from the surrounding liquid medium. A cation exchange resin has negative functional fixed ions and exchanges positive counter-ions.

The anchor groups (exchange-active components) in commercially available cation exchangers are usually -C₆H₅O⁻, -SO₃⁻, -COO⁻, -PO₃⁻, or -AsO₃⁻. Weaker cation exchange resins are those in which the binding strength of the cation is not high, such as those with carboxyl or carboxyalkyl functionalities. Furthermore, weaker cation exchange resins are usually not fully dissociated at acidic pH. A particular weak cation exchange resin used in the invention is comprised of a matrix of silica particles covalently bound to polyethyleneimine silane, wherein the amino groups of the polyethyleneimine have been derivatized with carboxyl groups. Such a resin is commercially available from J.T. Baker, under the trade name Widepore CBX® chromatography resin.

"Hydrophobic interaction resin" refers to a natural or artificial substance, usually a solid, which contains uncharged groups, such as methyl, ethyl, or other alkyl groups. These groups form hydrophobic bonds with groups on protein moieties which are passed through the resin and result in separation of proteins based on the strength of interaction between the protein and resin groups. A particular hydrophobic interaction resin is composed of semi-rigid spherical beads synthesized by a copolymerization of ethylene glycol and methacrylate type polymers derivatized with butyl groups. Such a resin is commercially available from Toso-Haas, under the trade name Toyo-Pearl® 650M C4.

"Affinity chromatography resin" refers to a natural or artificial substance, usually a solid, which is used for the purification of proteins. The resin separates proteins based on the affinity which occurs between groups on the protein and groups on the resin. In the instant invention, the resin used as an affinity chromatography resin is usually used as a size exclusion resin to separate proteins based on their size. A particular affinity chromatography resin is a cross-linked co-polymer of allyl dextran and N,N'-methylene bisacrylamide in the form of beads which are capable of fractionating globular proteins between 20,000 and 8,000,000 kDa. Such a resin is commercially available from Pharmacia, under the trade name of Sephacryl® S-400.

"Alkyl" refers to a straight or branched chain monovalent radical consisting solely of carbon and hydrogen, containing no unsaturation and having from one to eighteen carbon atoms, preferably from one to six carbon atoms, *e.g.* methyl, ethyl, *n*-propyl, isopropyl (1-methylethyl), *n*-butyl, *t*-butyl (1,1-dimethylethyl), *sec*-butyl (1-methylpropyl), *n*-pentyl, *n*-hexyl, and the like.

"Pure" as applied to the purity of the rDSPA α1 product following the purification scheme detailed in this application means that greater than 80% of the total protein in the final purification product is rDSPA α1, preferably greater than 90% of the total protein isolated is rDSPA α1 and most preferably 98% of the total protein isolated is rDSPA α1. Protein content and purity are based on reverse phase HPLC and SDS-page gel analysis.

"Non-rDSPA α1 protein and non-protein contaminants" refers to all material other than rDPSA α1 found within the biological media from which rDSPA α1 is being purified.

"Pharmaceutically acceptable excipient" refers to an acceptable carrier, and any pharmaceutically acceptable auxiliary substance as required to be compatible with physiological conditions, which are non-toxic and do not adversely effect the biological activity of the pharmaceutical composition suspended or included within it. Suitable excipients would be compounds such as mannitol, succinate, glycine, or serum albumin.

"Therapeutically effective amount" refers to that amount of rDSPA α1 which, when administered to a human in need thereof, is sufficient to effect treatment, as defined below, for disease-states characterized by thrombosis. The amount of a compound which constitutes a "therapeutically effective amount" will vary depending on the compound, the disease-state and its severity, but can be determined routinely by one of ordinary skill in the art having regard to his own knowledge and to this disclosure.

"Treating" or "treatment" as used herein cover the treatment of a disease-state in a human, which disease-state is characterized by thrombosis, and include:
(i) preventing the disease-state from occurring in a human, in particular when such human is predisposed to the disease-state but has not yet been diagnosed as having it;
(ii) inhibiting the disease-state, i.e., arresting its development; or
(iii) relieving the disease-state, i.e., causing regression of the disease-state.

### Description of the Preferred Embodiments

The present invention is directed to a method for the isolation and purification of rDSPA α1 on a commercial scale and in a form suitable for use in pharmaceutical formulations. The rDSPA α1 is produced by fermenting a mammalian cell line capable of secreting the rDSPA α1 product into the culture media. The conditioned media, i.e. that media obtained from the bioreactor containing the mammalian cells, is harvested, and the rDSPA α1 is separated from other proteins and contaminants by a series of chromatographic steps, beginning with the use of a cation exchange resin, followed by selective elution of the rDSPA α1 from the resin. The rDSPA α1 fraction obtained by selective elution from the cationic exchange resin is then applied to a hydrophobic interaction resin, where a selective elution from the matrix provides a second level of purification. The eluted rDSPA α1 fraction is then applied to an affinity chromatography resin, where selective elution provides a further level of purification. The purified rDSPA α1 is next concentrated by conventional techniques, such as ultrafiltration, and then lyophilized.

Specifically, the invention is practiced as described below.

### A. Isolation and Purification

### 1. Culture Media and Cell Lines

The culture medium comprises a base medium suitable for mammalian cell growth, such as DMEM or Ham's F12. A particular medium is William's E medium (Williams, G.M. and Gunn, J.M, *Exp*. *Cell Res*., (1974) 89:139). For the inoculation growth phase, the base medium will usually be supplemented with a serum source, typically bovine serum (BS) or newborn calf serum (CS), present at a concentration in the range from about 0.1% to 10% by weight, usually being present at about 1% to 5% by weight. Other growth factors or buffers, such as HEPES, may also be added. During the perfusion growth phase, the serum concentration is usually maintained at the same concentration, typically being in the range from about 3% to 8%, usually being about 5%.

Cell lines suitable for use in the present invention include mammalian cell lines capable of non-adherent growth in suspension culture and/or adherent growth on microcarrier beads. Particular cell lines which meet these requirements include Chinese hampster ovary (CHO) cell lines, BHK cells, or the HEK293 cell line (Krätzschmer et al., *Gene,* (1991) 116: 281-284; Petri, T, J. *BioTechnology,* (1995) 39: 75-83).

A particularly preferred CHO cell line is DXB11, which is described in Urlaub, G. and Chasin, LA, Proc. *Natl. Acad. Sci. USA,* (1980) 77: 4216-4220. These cells have been co-transfected with the expression vectors pSVPA11 and pUDHFR1, which contain the coding sequences for DSPA α1 and mouse dihydrofolate reductase, respectively (Petri, T., ibid). The transformed CHO cell line used in the present invention is designated CD16 and has been deposited with the American Type Culture Collection, Rockville, Maryland (ATCC) and have been given ATCC # CRL 12023.

### 2. Expansion of Culture and Production Phase

After inoculation with either confluent spinner cultures or portions of other bioreactor cultures, the cell culture will be expanded to production density, typically in the range from about 1-40x10⁶ cells/ml.

After the desired cell density on microcarrier beads is achieved, perfusion of fresh media supplemented with serum is initiated. Alternatively, cells may be grown in suspension culture. Typically, the concentration of the serum in the fresh media will be in the range from about 2% to 10% by weight, more typically in the range from about 3% to 8% by weight, and more normally being about 5% by weight. Initially, the perfusion rate will be in the range from about 0.25 to 0.75 culture volumes/day, typically being about 0.5 culture volumes/day. As the cell growth increases, the perfusion rate is increased to a final rate in the range from about 1.5 to 2.5 culture volumes/day, typically over a period of about 2 to 10 days. During the expansion, sterile, pre-equilibrated microcarrier beads are added to the reactor to maintain the microcarrier bead to cell density ratio in the range from about 0.5 to 1.0 grams of microcarrier beads to 10⁹ cells. Conveniently, the microcarrier beads are added to the reactor using an aspirator through the sample line.

After cell density has reached the production level, the serum addition to the fresh culture medium will be reduced, typically to a concentration in the range from about 0.1 to 2.0 weight percent, typically 1%.

The culture in production phase requires attention to multiple fermentation parameters: temperature, pH, and the level of dissolved oxygen are monitored daily. Additional culture media, serum, and alkali need to be provided as the supply tanks are depleted. Samples of the conditioned media, defined as media which contains product, are analyzed routinely, at least every two days to assure that production continues free from contamination.

The procedure for collection of the conditioned media from bioreactors minimizes the harvesting of cells by using screens with approximately 100-150 micron pore diameters. Suspension cultures use a vortex flow fitter to retain cells in the bioreactor. The harvest is collected in sterile containers and stored for up to 38 days before further processing. rDSPA α1 found in the bioreactor harvest has been secreted from the CHO cells in a processed form which has full biological activity and is ready for purification.

### 3. Cation Exchange Chromatography

After pH adjustment to the range from 4 to 6, preferably to about 5, the rDSPA α1 in the conditioned media is applied to a cation exchange matrix (usually packed in the form of a column) under conditions selected to provide essentially complete binding of rDSPA α1 to the matrix. While other proteins will also be bound, the initial binding stage provides a first level of separation as a number of the undesired or contaminating proteins and other compounds, such as phenol red, in the conditioned media will be unable to bind to the matrix and thus will flow through the matrix. The rDSPA α1 is further purified by selective elution from the matrix, where the elution may be accomplished by either a stepwise edition or linear gradient elution by increasing the ionic strength of the buffer. In either case, the rDSPA α1 is collected for further purification as described below.

Suitable cation exchange matrices include a wide variety of resins derivatized with cationic functionalities which are able to bind rDSPA α1. Preferred are synthetic resins, such as those comprised of silica gel particles, cross-linked agarose, or cross-linked polymethacrylate polymers, derivatized with cationic functionalities such as carboxyl, carboxymethyl, sulfonyl, phosphoryl, and the like. Particularly useful are relatively weak resins, such as those having carboxyl or carboxyalkyl functionalities, such as carboxymethyl or carboxyethyl. A particularly preferred resin is comprised of a matrix of silica particles covalently bound to polyethyleneimine silane, with amino groups of the polyethyleneimine silane derivatized with carboxyl groups. Such a resin is Baker Widepore CBX® (45mm bead size), which is commercially available from J.T. Baker.

The binding and elution conditons will vary depending on the binding strength of the cationic resin. For weak cationic resins, such as Baker Widepore CBX, binding may be effected at low ionic strength under slightly acidic conditions, typically pH 4-7, preferably about pH 5. After washing the matrix, the rDSPA α1 may be selectively eluted by exposing the matrix to a mobile phase having an elevated ionic strength, employing either linear or stepwise elution. For the Baker Widepore CBX resin, the rDSPA α1 will elute at a pH of about 7.5, with a salt concentration between about 100mM and 500mM NaCI. The column may then be stripped and regenerated for subsequent use.

Preferrably, with the Baker Widepore CBX matrix, the resin will initially be equilibrated with a buffer of 100mM sodium acetate, pH 5. Buffer is applied to the column at a flow rate of 0.2 to 2.0 column volumes per minute until the pH of the effluent stabilizes at 5. The conditioned media containing rDSPA α1 is filtered with a 1.2 um filter and then titrated to a pH between 4 and 7, most preferrably 5, using glacial acetic acid. The media is then applied to the column, typically using a gear pump, at a flow rate no greater than 2.0 column volumes per minute. A filter is provided to remove particulates which might plug the column matrix.
The column matrix is then re-equilibrated with the acetate equilibration buffer until the pH stabilizes at 5, typically requiring from about 2 to 7 column volumes. A wash buffer containing 50mM sodium phosphate, pH 7.5 is next applied to the column at about 0.1 to 0.2 column volumes per minute until the pH of the eluent stabilizes at 7.5. rDSPA α1 is then eluted from the column using an elution buffer containing 50mM sodium phosphate, 500mM sodium chloride, pH 7.5. The elution buffer is run until no more protein is found eluting from the column. A stripping buffer containing 2.0 M sodium acetate, pH 8 is then applied to the column in order to regenerate the matrix. The storage buffer contains 10% acetic acid and 45% ethanol.

### 4. Hydrophobic Interaction Chromatography

The rDSPA α1 fraction collected from the cation exchange matrix is next applied to a hydrophobic interaction matrix (usually in the form of a column) under conditions which allow binding of the rDSPA α1 to the matrix, typically high ionic strength and low pH. The rDSPA α1 is then selectively eluted by increasing the organic solvent concentration of a mobile phase applied to the column, using a linear or a step-wise gradient. The rDSPA α1 fraction is collected for further purification. This step reduces DNA contamination by 100 to 1000 fold and inactivates potential viral contaminants.

Suitable hydrophobic interaction matrices include a wide variety of uncharged resins having covalently attached hydrophobic groups, such as propyl, butyl, octyl, phenyl, and the like. The resins may be cross-linked organic polymers, such as styrene-divinylbenzene, silica, agarose, polymethacrylate, or any one of a wide variety of other suitable particulate supports. A particularly preferred resin is comprised of semi-rigid spherical beads synthesized by a copotymerization of ethylene glycol and methacrylate type polymers derivatized with butyl groups. Such a resin is Toyo-Pearl® 650M (40-90µm beads) which is commercially available from Toso-Haas.

Binding to the hydrophobic interaction column is effected under conditions of high ionic strength, usually at an acidic pH from 3-5, more usually about 4. Substantially all the protein contained in the rDSPA α1 fraction which had been eluted from the cation exchange resin is bound to the hydrophobic interaction column. The various proteins may be selectively eluted based on the differing strengths of hydrophobic interaction with the hydrophobic groups on the matrix, i.e., in order of increasing hydrophobicity of the protein. Elution may be performed with a step-wise or linear gradient, usually with an alcohol eluant, such as ethanol or isopropanol. A particularly preferred alcohol is ethyl alcohol.

Preferrably, with the Toyo-Pearl C4 matrix, equilibration may be performed with an equilibration buffer having 50mM sodium acetate, 500mM sodium chloride, pH 4. After the rDSPA α1 fraction from the ion exchange column is titrated to pH 4, it is applied to the C4 matrix, and the matrix is then re-equilibrated with the equilibration buffer described above. The column is then washed sequentially with two buffers, as follows. The first wash (Wash 1) uses at least two column volumes of a buffer containing 20mM HCL Washing is continued until the effluent contains no further protein, as evidenced by a steady UV absorbance. The matrix is then washed (Wash 2) with not less than 10 column volumes of a buffer containing 19% ethanol, 20mM HCI, and washing is continued with a buffer containing 20.5% ethanol, 20 mM HCI until no further protein is eluted. Elution of the rDSPA α1 product is effected using a buffer containing 29% ethanol, 20mM HCl, pH 2.5. After eluting the product, the column is stripped with not less than two column volumes of a buffer containing 100mM NaOH. The matrix is stored in Wash 1 buffer.

### 5. Affinity chromatography

The rDSPA α1 fraction collected from the hydrophobic interaction resin is next applied to an affinity matrix (usually in the form of a column) under conditions which allow binding of the rDSPA α1 to the affinity matrix. While the rDSPA α1 remains bound to the column, contaminants are eluted by washing the column with 2 to 3 column volumes of 20 mM HCI. This step facilitates a buffer exchange to aid pharmaceutical formulation and solubility and also helps in the inactivation/removal of potential viral contaminants. Preferably, 2.5 column volumes of the buffer is used. The rDSPA α1 is then selectively eluted using a buffer containing 200mM glycine, free base at a pH between 4 and 5. The rDSPA α1 fraction is collected for concentration.

In this invention, the resins used as affinity resins are ones which are normally used as size exclusion resins. Suitable affinity matrices indude resins comprised of a cross-linked polymer of allyl dextran and N.N'-methylene bisacrylamide in the form of beads with a diameter between 25 and 75 µm. A particularly preferred resin is Sephacryl 400®, which is commercially available from Pharmacia and is capable of fractionating globular proteins between 20,000 and 8,000,000 kDa.

Binding of rDSPA α1 to the affinity resin is achieved under conditions of low ionic strength and low pH. Substantially all the rDSPA α1 collected from the hydrophobic interaction resin is bound to the column. The rDSPA α1 may be selectively eluted by raising the pH and/or ionic strength. Elution may be performed with a step-wise or linear gradient, usually with an eluent containing 200mM glycine, free base.

Preferrably, with the Sephacryl S-400 matrix, equilibration may be performed with a buffer having 19% ethanol, 20mM HCl, until the pH of the effluent remains unchanged. The matrix is loaded with the rDSPA α1 fraction from the hydrophobic interaction column, and then washed with a buffer containing 20mM HCl until the UV signal from the effluent is steady. Elution of bound rDSPA α1 is effected using a buffer containing 200mM glycine. After eluting the product, the column is stripped with not less than two column volumes of a buffer containing 100mM NaOH. Following washing of the matrix with at least two column volumes of the wash buffer (20mM HCI), the matrix can be stored in this buffer.

### 6. Concentration

The purified protein of the present invention may then be concentrated, typically by filtration, or the like, and may eventually be lyophilized or otherwise incorporated into conventional pharmaceutical preparations. Useful concentration and lyophilization methods are well described in the scientific literature.

### B. Formulation

### 1. Pharmaceutical compositions

This invention provides a reagent with significant therapeutic value. rDSPA α1, purified by the methods described, which should be useful in the treatment of arterial and venous thromboses.

Recombinant DSPA α1, purified as described herein, can be administered to a patient This reagent can be combined for therapeutic use with other ingredients, e.g., in conventional pharmaceutically acceptable carriers or diluents, along with physiologically innocuous stabilizers and excipients. These combinations can be sterile filtered and placed into dosage forms as by lyophilization in dosage vials or storage in stabilized aqueous preparations.

The quantities of reagent necessary for effective therapy will depend upon many different factors, including means of administration, physiological state of the patient, and other medicants administered. Thus, treatment dosages should be titrated to optimize safety and efficacy. Typically, dosages used *in vitro* may provide useful guidance in the amounts useful for *in situ* administration of these reagents. Animal testing of effective doses for treatment will provide further predictive indication of human dosage. Various consideration are described, e.g., in Gilman et al. (eds) (1990) *Goodman and Gilman's: The Pharmacological Bases of Therapeutics,* 8th.Ed., Pergamon Press; and *Remington's Pharmaceutical Sciences*, 18th ed. (1990), Mack Publishing Co., Easton, Penn.;

Methods for administration are discussed therein and below, e.g., for oral, intravenous, intraperitoneal, or intramuscular administration, transdermal diffusion, and others. Pharmaceutically acceptable carriers will indude water, saline, buffers, and other compounds described, e.g., in the Merck Index, Merck & Co., Rahway, New Jersey. Based on the dosages required of other plasminogen activators, the need for a total dosage of between 80 and 110 mg would be expected *(Physicians' Desk Reference,* 49th ed. (1995), Medical Economics Data Production Company, pp 1083-1085).

Therapeutic formulations may be administered in any conventional dosage formulation. While it is possible for the active ingredient to be administered alone, it is preferable to present it as a pharmaceutical formulation. Formulations comprise at least one active ingredient, as defined above, together with one or more acceptable carriers thereof. Each carrier must be both pharmaceutically and physiologically acceptable in the sense of being compatible with the other ingredients and not injurious to the patient. Formulation include those suitable for oral or parenteral (including subcutaneous, intramuscular, intravenous and intradermal) administration. The formulations may conveniently be presented in unit dosage form and may be prepared by any methods well known in the art of pharmacy. See, e.g. Gilman et. al., ibid, and Remington ibid..

In summary, the preferred embodiments of the invention as described in the Summary of the Invention are as follows:
a method for the isolation and purification of rDSPA α1 from a biological medium, the method comprising the following steps:
   (a) applying the medium at a pH of 5 to a cation exchange resin comprised of a matrix of silica particles covalently bound to polyethyleneimine silane, wherein the amino groups have been derivatized with carboxyl groups ;
   (b) washing the cation exchange resin at pH 5 with 100mM NaOAc and 50 mM NaPO₄ at pH 7.5, to remove non-rDSPA α1 protein and non-protein contaminants;
   (c) eluting the bound rDSPA α1 from the cation exchange resin with a buffer containing 50 mM sodium phosphate and 500 mM sodium chloride at pH 7.5;
   (d) applying the rDSPA α1-containing eluent from step (c) at pH 4 to a hydrophobic interaction resin comprised of semi-rigid spherical beads synthesized by a copolymerization of ethylene glycol and methacrylate type polymers derivatized with butyl groups;
   (e) washing the hydrophobic interaction resin with 20 mM HCl and then with 20 mM HCI containing 19% EtOH to remove non-rDSPA α1 protein and non-protein contaminants;
   (f) eluting the bound rDSPA α1 from the hydrophobic interaction resin with a buffer containing 29% ethanol and 20 mM hydrochloric acid at pH 2.5;
   (g) applying the rDSPA α1-containing eluent from step (f) at pH 2.5 to an affinity chromatography resin comprised of a cross-linked polymer of allyl dextran and N,N'-methylene bisacrylamide which fractionates globular proteins between 20,000 and 8,000,000 kDa;
   (h) washing the affinity chromatography resin with 20 mM HCI to remove non-rDSPA α1 protein and non-protein contaminants;
   (i) eluting the bound rDSPA α1 from the affinity chromatography resin with a buffer containing 200 mM glycine at a pH between 4 and 5, to produce pure rDSPA α1 in an aqueous solution.

The following specific preparations and examples are provided as a guide to assist in the practice of the invention, and are not intended as a limitation on the scope of the invention.

### EXAMPLE 1

### Cell Culture Production of rDSPA α1

A vial from the DSPA Working Cell Bank was thawed and inoculated into a roller bottle with 5% calf serum in growth media (WEC 5.0 media, Williams E modified). Over a two week period the cells were expanded into four roller bottles, the cells were trypsinized from the roller bottles and inoculated into four 1L spinner flasks at 4 x 10⁸ cells each in 1L of growth media. After four days, the cultures from the four spinners were used to inoculate a 10L bioreactor. An additional 1L of growth media was added to the bioreactor culture on the day of inoculation. The next day, the bioreactor was filled to 10L with growth media. Culturing both with and without addition of microcarrier beads followed this protocol. The medium pH was controlled at 7.0-7.4 and oxygenation was maintained at all times by a sparging system. The temperature was maintained at 34-39°C.

The cell density on the day of inoculation in the bioreactor was 8.1 x 10⁵ cells per mL and after two days the cell density had doubled, at which time the perfusion rate was set at 0.5 culture volumes per day (cv/day). The perfusion rate was increased in relation to cell density such that by nine days post inoculation of the bioreactor the density had reached 6.2 x 10⁶ cells/mL and perfusion increased to 2 cv/day.

The bioreactor was then switched to production media (1% calf serum in WEC 5.0 media) and two days later collection of production harvest began and continued for as long as 10 weeks. During the production phase, the average cell density was 15 x 10⁶ cells per mL with approximately 75% viability. The average rDSPA α1 production was 40 milligrams rDSPA α1 per liter and average specific production rate was 6 picograms rDSPA α1 per cell per day.

Expansion of fermentation volumes was achieved by transferring half the contents of one reactor to a new vessel. Both bioreactors were placed in production media, perfused at 2 cv/day, and collection of production harvest began immediately.

### EXAMPLE 2

### Purification of rDSPA α1

1. Cation Exchange Chromatography (Column A). The bioreactor harvest is stored at ambient temperature (15-25"C). It is then filtered through a 0.45 micron filter prior to loading onto a cation exchange column. Purification of the harvest begins with a column chromatography step using the CBX resin made by J.T. Baker (Column A). This step facilitates a significant purification of the protein.
   The Column A buffers consist of Buffer A1: Equilibration buffer (50mM NaoAc, pH 5.0), Buffer A2: Wash buffer (50mM NaPO₄, pH 7.5), Buffer A3: Elution buffer (50mM NaPO₄, 500 mM NaCl, pH 7.5), Buffer A4: Strip buffer (2 M NaAc, pH 8.0), and Buffer A5: Storage buffer (45% EtOH, 10% HoAc).
   The following column operating parameters are appropriate for a column packed with 6 kg of resin and has a column volume of 15 liters. Not more than 4.5 grams of rDSPA α1 can be loaded on the column per run. The column is loaded at a flow rate of not more than 2 liters per minute and not more than 20 psi column pressure. The column and monitor specifications are checked prior to each run. The load material and chromatographic buffers are degassed by sparging with helium prior to use.
   The bioreactor harvest is filtered with a 1.2 mm filter then titrated to pH 5.00 (± 0.10) using glacial acetic acid. Prior to loading, the column is equilibrated with not less than 30 liters of Buffer A1 until the effluent is pH 5.00 (± 0.20). Once the column is equilibrated, the harvest is loaded on the column. The column is re-equilibrated with not less than 80 liters of Buffer A1. The column is properly re-equilibrated when the effluent is pH 5.00 (± 0.20) and when a stable UV baseline is reached. The column is washed with not less than 145 liters of Buffer A2. The column is properly washed when the effluent is pH 7.50 (t 0.20) and when a stable UV baseline is reached.
   The product is eluted from the column with not less than 30 liters of Buffer A3 and collected in a separate vessel. Elution is considered complete when a stable UV baseline is reached. After eluting the product, the column is stripped until a stable UV baseline is achieved, using not less than 30 liters of Buffer A4. The column is then cleaned for reuse (not to exceed 50 cycles of use). The Column A product (or eluate) is stored at 2-8°C. The average recovery was 93% and the average (protein) purity of the eluate was 81%.
2. Hydrophobic Interaction Chromatography (Column B). Following chromatography on the CBX resin, the rDSPA α1 product is chromatographed using a C4 hydrophobic interaction resin (Toso-Haas) (Column B). This step facilitates significant removal of non-protein contaminants and also helps in the inactivation/removal of possible viral contaminants. The Column B buffers consist of Buffer B1: Equilibration buffer (50mM NaoAc, 500mM NaCI, pH 4.0), Buffer B2: Wash and Storage buffer (20mM HCl), Buffer B3: Wash buffer (20mM HCl, 19% EtOH), Buffer B4: Wash buffer (20mM HCl, 20.5% EtOH), Buffer B5: Elution buffer (20mM HCI, 29.5% EtOH), and Buffer B6: Strip buffer (0.1N NaOH). The following column operating parameters are appropriate for a column volume of 15 liters. Not more than 9 grams of rDSPA α1 can be loaded on the column per run. The column is loaded at a flow rate of not more than 2 liters per minute and not more than 15 psi column pressure. The column and monitor specifications are checked prior to each run.
   Prior to loading, the column is equilibrated until the effluent is pH 4.00 (± 0.20). The Column A eluate is titrated to pH 4.00 (± 0.10) using glacial acetic acid, degassed, and loaded. This column is re-equilibrated with not less than 30 liters of Buffer B1 until the effluent is pH 4.00 (± 0.20) and a stable UV baseline is reached. The column is washed with three buffers. The first wash is with not less than 45 liters of Buffer B2. The column is property washed when the effluent is pH 1.90 (± 0.20) and when a stable UV baseline is reached. The column is washed a second time with not less than 145 liters of Buffer B3 until a stable UV baseline is reached. The column is washed a third time with not less than 30 liters of Buffer B4 and is complete when a stable UV baseline is reached.
   The rDSPA α1 is eluted from the column with not less than 30 liters of Buffer B5 and collected in a separate vessel. Elution is continued until a stable UV baseline is reached. The column is then cleaned for reuse (not to exceed 50 cycles of use). The Column B product (or eluate) is stored at 2-8°C for not more than 15 days before further processing. The average recovery was 91% and the average (protein) purity of the eluate was 97%.
3. Affinity chromatography (Column C). The Column B product is next chromatographed using Sephacryl S-400 (Pharmacia) (Column C). This step facilitates a buffer exchange to aid formulation and also helps in the inactivation/removal of viral contaminants. The Column C buffers consist of Buffer C1: Equilibration buffer (20mM HCI, 19% EtOH), Buffer C2: Wash buffer (20mM HCl), Buffer C3: Elution and Storage buffer (200mM glycine, sterile filtered), and Buffer C4: Strip buffer (0.1 N NaOH). The following column operating parameters are appropriate for a column volume of 10 liters. Not more than 20 grams of rDSPA α1 can be loaded on the column per run. The column is loaded at a flow rate of not more than 0.5 liters per minute and not more than 15 psi column pressure. The eluates from one or more Column B runs are pooled, then diluted with one part Buffer C2 and two parts Buffer 5 eluate. The final ethanol concentration will be approximately 19%.

Prior to loading, the column is equilibrated with not less than 15 liters of Buffer C1 until the effluent is pH 1.80 (± 0.20). After loading the column is washed with not less than 30 liters of Buffer C2. The column is properly washed when the UV signal is stable. The product is eluted from the column with not less than 20 liters of Buffer C3 and collected in a separate vessel. Elution is continued until a stable UV baseline is reached.

After eluting the product, the column is stripped with not less than 12 liters of Buffer C4 and stored until reuse not to exceed 20 cycles. The Column C product (or eluate) is diluted to a concentration of < 1 mg per milliliter with Buffer C3 and stored at 2-8°C for further processing. The average recovery of rDSPA α1 was 97% and the average (protein) purity of the eluate was 98%.

The concentration and formulation steps followed the completion of the purification steps above and was performed on the S-400 affinity column eluate, which had been stored for not more than 70 days. Column C eluates were pooled and concentrated by a spiral-wound ultrafiltration membrane that removes low molecular weight substances (30,000 dalton cut-off). The product was collected into a pyrogen free container at a concentration greater than
8 mg/mL Mannitol was added to a 4% final concentration.

Final formulation buffer (200mM glycine, 4% mannitol (w/v) was added to bring the concentration of the bulk formulated product to 7.5mg/mL The bulk formulated product is then sterile filtered, dispensed into vials and lyophilized.

## Claims

1. A method for purifying recombinant Desmodus rotundus salivary plasminogen activator (rDSPAα1) from a biological medium, the method comprising the following:
(a) applying the medium to a cation exchange resin at a pH between 4 and 7 ;
(b) washing the cation exchange resin to remove non-rDSPA α1 proteins and non-protein contaminants;
(c) selectively eluting the bound rDSPA α1 from the cation exchange resin;
(d) applying the rDSPA α1-containing eluent from step (c) to a hydrophobic interaction resin at a pH between 3 and 5;
(e) washing the hydrcchobic interaction resin to remove non-rDSPA α1 protein and ncn-protein contaminants;
(f) selectively eluting the bound rOSPA α1 from the hydrophobic interaction resin;
(g) applying the rDSPA α1-containing eluent from step (f) to an affinity chromatography resin at low pH and low ionic strength;
(h) washing the affinity chromatography resin to remove non-rDSPA α1 protein and non-protein contaminants;
(i) selectively eluting the bound rOSPA α1 from the affinity chromatography resin to produce pure rDSPA α1 in an aqueous solution.

2. The method of Claim 1. wherein the biological medium is a conditioned medium.

3. The method of Claim 1, wherein the ration exchange resin in step (a) is comprised of silica gel particles, cross-linked agarose. or cross-linked polymethacrylate polymers, derivatized with carboxyl or carboxyalkyl groups.

4. The method of Claim 3, wherein the cation exchange resin is comprised of a matrix of silica particles covalently bound to polyethyleneimine silane, wherein the amino groups of the polyethyleneimine silane have been derivatized with carboxyl groups.

5. The method of Claim 3, wherein the loading conditions in step (a) include applying the media at a pH between 4 and 7.

6. The method of Claim 4, wherein the elution of rDSPA α1 in step (c) is performed using a buffer containing 50 mM NaPhos and between 100 mM and 500 mM NaCI, or a buffer of equivalent ionic strength.

7. The method of Claim 1, wherein the hydrophobic interaction resin in step (d) is an uncharged resin, derivatized with alkyl chains of 1-10 carbons in length or with aryl-alkyl groups.

8. The method of Claim 7, wherein the uncharged resin is comprised of silica gel particles, cross-linked agarose, or a cross-linked polymethacrylate polymer.

9. The method of Claim 7, wherein the uncharged resin is comprised of semi-rigid spherical beads synthesized by a copolymerization of ethylene glycol and methacrylate type polymers, derivatized with butyl groups.

10. The method of Claim 9, wherein the loading conditions in step (d) include applying the eluent from step (c) at a pH between 3 and 5.

11. The method of Claim 9, wherein the loading conditions in step (d) include applying the eluent from step (c) in 50 mM NaPhos, 500 mM NaCI, pH adjusted to 4 with phosphoric acid, or in a buffer of equivalent ionic strength

12. The method of Claim 9, wherein the elution in step (f) is performed using a buffer containing 20 mM HCl and having an ethanol concentration greater than 25%.

13. The method of Claim 12, wherein the ethanol concentration is between 28.5% and 30%.

14. The method of Claim 12, wherein the ethanol concentration is 29%.

15. The method of Claim 1, wherein the affinity chrcmatography resin of step (g) is comprised of a cross-linked co-polymer of allyl dextran and N.N'-methylene bisacrylamide in the form of beads with a diameter between 25 and 75 µm.

16. The method of Claim 15, wherein the beads are capable of fractionating globular proteins between 20,000 and 8.000,000 kDa.

17. The method of Claim 15, whefein the loading conditions in step (g) include applying the eluent from step (f) at a pH between 1 and 4.

18. The method of Claim 15. wherein the elution in step (i) is performed using a buffer containing 200 mM glycine at a pH between 3 and 5, or a buffer of equivalent ionic strength.

19. The method of Claim 1, further comprising concentrating the aqueous solution of rDSPAα1.

20. The method of Claim 19, further comprising lyophilizing the concentrated rDSPA α1 solution.

21. The method of Claim 1, further comprising concentrating the aqueous solution of rDSPA α1 and lyophilizing the concentrated rDSPA α1 solution.

22. The method of Claim 1, wherein the method comprises the following steps:
(a) applying the medium at a pH between 4 and 7, to a cation exchange resin comprised of silica gel particles, cross-linked agarose, or cross-linked polymethacrylate polymers, derivatized with carboxyl or carboxyalkyl groups;
(b) washing the cation exchange resin to remove non-rDSPA α1 proteins and non-protein contaminants;
(c) selectively eluting the bound rDSPA α1 from the cation exchange resin using a buffer containing 50 mM sodium phosphate and between 100 mM and 500 mM NaCl, or a buffer of equivalent ionic strength;
(d) applying the rDSPA α1-containing eluent from step (c) at a pH between 3 and 5 to a hydrophobic interaction resin comprised of silica gel particles, cross-linked agarose, or linked polymethacrylate polymers;
(e) washing the hydrophobic interaction resin to remove non rOSPA α1 protein and non-protein contaminants;
(f) selectively eluting the bound rDSPA α1 from the hydrophobic interaction resin using a buffer containing 20 mM HCl and having an ethanol concentration greater than 25%:
(g) applying the rDSPA α1-containing eluent from step (f) at a pH between 1 and 4 to an affinity chromatography resin comprised of a cross-linked polymer of allyl dextran and N,N'-methylene bisacrylamide in the form of beads with a diameter between 25 and 75 um;
(h) washing the affinity chromatography resin to remove non-rDSPA α1 protein and non-protein contaminants;
(i) selectively eluting the bound rDSPA α1 from the affinity chromatography resin with a buffer containing 200 mM glycine at a pH between 3 and 6 or a buffer of equivalent ionic strength, to produce pure rDSPA α1 in an aqueous solution.

23. A method for the isotation and purification of rDSPA α1 from a biological medium, the method comprising the following steps:
(a) applying the medium at a pH of 5 to a cation exchange resin comprised of a matrix of silica particles covalently bound to polyethyleneimine silane, wherein the amino groups have been derivatized with carboxyl groups;
(b) washing the cation exchange resin at pH 5 with 100mM NaOAc and 50 mM NaPO₄ at pH 7.5, to remove non-rDSPA α1 protein and non-protein contaminants;
(c) eluting the bound rDSPA α1 from the cation exchange resin with a buffer containing 50 mM sodium phosphate and 500 mM sodium chloride at pH 7.5;
(d) applying the rDSPA α1-containing eluent from step (c) at pH 4 to a hydrophobic interaction resin comprised of semi-rigid spherical beads synthesized by a copolymerization of ethylene glycol and methacrylate type polymers derivatized with butyl groups;
(e) washing the hydrophobic interaction resin with 20 mM HCl and then with 20 mM HCl containing 19% EtOH to remove non-rDSPA α1 protein and non-protein contaminants;
(f) eluting the bound rDSPA α1 from the hydrophobic interaction resin with a buffer containing 29% ethanol and 20 mM hydrochloric acid at pH 2.5;
(g) applying the rDSPA α1-containing eluent from sfep (f) at pH 2.5 to an affinity chromatography resin comprised of a cross-linked polymer of allyl dextran and N,N'-methylene bisacrylamide which fractionates globular proteins between 20,000 and 8,000,000 kDa;
(h) washing the affinity chromatography resin with 20 mM HCl to remove non-rDSPA α1 protein and non-protein contaminants;
(i) eluting the bound rDSPA α1 from the affinity chromatography resin with a buffer containing 200 mM glycine at a pH between 4 and 5, to produce pure rDSPA α1 in an aqueous solution.

## Patentansprüche

1. Verfahren zur Reinigung eines rekombinanten Desmodus rotundus Speichel-Plasminogenaktivators (rDSPAα1) aus einem biologischen Medium, wobei das Verfahren folgendes umfaßt:
(a) Auftragen des Mediums auf ein Kationenaustauscherharz bei einem pH-Wert zwischen 4 und 7;
(b) Waschen des Kationenaustauscherharzes zur Entfernung von Nicht-rDSPAα1-Proteinen und Nicht-Protein-Verunreinigungen;
(c) selektives Eluieren des gebundenen rDSPAα1 vom Kationenaustauscherharz;
(d) Auftragen des rDSPAα1-haltigen Eluats aus Schritt (c) auf ein Harz mit hydrophober Wechselwirkung bei einem pH-Wert zwischen 3 und 5;
(e) Waschen des Harzes mit hydrophober Wechselwirkung zur Entfernung von Nicht-rDSPAα1-Protein und Nicht-Protein-Verunreinigungen;
(f) selektives Eluieren des gebundenen rDSPAα1 vom Harz mit hydrophober Wechselwirkung;
(g) Auftragen des rDSPAα1-haltigen Eluats aus Schritt (f) auf ein Affinitätschromatographieharz bei einem niedrigen pH-Wert und einer niedrigen Ionenstärke;
(h) Waschen des Affinitätschromatographieharzes zur Entfernung von Nicht-rDSPAα1-Protein und Nicht-Protein-Verunreinigungen;
(i) selektives Eluieren des gebundenen rDSPAα1 vom Affinitätschromatographieharz zur Herstellung von reinem rDSPAα1 in einer wäßrigen Lösung.

2. Verfahren nach Anspruch 1, wobei es sich bei dem biologischen Medium um ein konditioniertes Medium handelt.

3. Verfahren nach Anspruch 1, wobei das Kationenaustauscherharz in Schritt (a) aus Kieselgelpartikeln, quervernetzter Agarose oder quervernetzten Polymethacrylatpolymeren, derivatisiert mit Carboxyl- oder Carboxyalkylgruppen, besteht.

4. Verfahren nach Anspruch 3, wobei das Kationenaustauscherharz aus einer Matrix aus Kieselgelpartikeln, die kovalent an Polyethyleniminosilan, dessen Aminogruppen mit Carboxylgruppen derivatisiert sind, gebunden sind, besteht.

5. Verfahren nach Anspruch 3, wobei die Beladungsbedingungen in Schritt (a) das Auftragen der Medien bei einem pH-Wert zwischen 4 und 7 umfassen.

6. Verfahren nach Anspruch 4, wobei die Elution von rDSPAα1 in Schritt (c) mit einem Puffer, der 50 mM Na-phosphat sowie zwischen 100 mM und 500 mM NaCl enthält, oder mit einem Puffer von äquivalenter lonenstärke durchgeführt wird.

7. Verfahren nach Anspruch 1, wobei es sich bei dem Harz mit hydrophober Wechselwirkung in Schritt (d) um ein ungeladenes, mit Alkylketten mit einer Länge von 1-10 Kohlenstoffatomen oder mit Arylalkylgruppen derivatisiertes Harz handelt.

8. Verfahren nach Anspruch 7, wobei das ungeladene Harz aus Kieselgelpartikeln, quervernetzter Agarose oder einem quervernetzten Polymethacrylatpolymer besteht.

9. Verfahren nach Anspruch 7, wobei das ungeladene Harz aus halbfesten kugelförmigen Perlen, die mittels Copolymerisation von Ethylenglykol und mit Butylgruppen derivatisierten Polymeren vom Methacrylat-Typ synthetisiert werden, besteht.

10. Verfahren nach Anspruch 9, wobei die Beladungsbedingungen in Schritt (d) das Auftragen des Eluats aus Schritt (c) bei einem pH-Wert zwischen 3 und 5 umfassen.

11. Verfahren nach Anspruch 9, wobei die Beladungsbedingungen in Schritt (d) das Auftragen des Eluats aus Schritt (c) in 50 mM Na-phosphat, 500 mM NaCl, mit einem mit Phosphorsäure eingestellten pH-Wert von 4, oder in einem Puffer von äquivalenter lonenstärke umfassen.

12. Verfahren nach Anspruch 9, wobei die Elution in Schritt (f) unter Verwendung eines Puffers mit 20 mM HCl und mit einer Ethanolkonzentration von mehr als 25% durchgeführt wird.

13. Verfahren nach Anspruch 12, wobei die Ethanolkonzentration zwischen 28,5% und 30% liegt.

14. Verfahren nach Anspruch 12, wobei die Ethanolkonzentration 29% beträgt.

15. Verfahren nach Anspruch 1, wobei das Affinitätschromatographieharz aus Schritt (g) aus einem quervernetzten Copolymer aus Allyl-Dextran und N,N'-Methylen-bisacrylamid in Perlenform mit einem Perlendurchmesser zwischen 25 und 75 µm besteht.

16. Verfahren nach Anspruch 15, wobei die Perlen in der Lage sind, globuläre Proteine zwischen 20 000 und 8 000 000 kDa aufzutrennen.

17. Verfahren nach Anspruch 15, wobei die Beladungsbedingungen in Schritt (g) das Auftragen des Eluats aus Schritt (f) bei einem pH-Wert zwischen 1 und 4 umfassen.

18. Verfahren nach Anspruch 15, wobei die Elution in Schritt (i) unter Verwendung eines Puffers mit 200 mM Glycin bei einem pH-Wert zwischen 3 und 6 oder eines Puffers von äquivalenter lonenstärke durchgeführt wird.

19. Verfahren nach Anspruch 1, weiterhin umfassend das Konzentrieren der wäßrigen rDSPAα1-Lösung.

20. Verfahren nach Anspruch 19, weiterhin umfassend das Lyophilisieren der konzentrierten rDSPAα1-Lösung.

21. Verfahren nach Anspruch 1, weiterhin umfassend das Konzentrieren der wäßrigen rDSPAα1-Lösung sowie das Lyophilisieren der konzentrierten rDSPAα1-Lösung.

22. Verfahren nach Anspruch 1, wobei das Verfahren die folgenden Schritte umfaßt:
(a) Auftragen des Mediums bei einem pH-Wert zwischen 4 und 7 auf ein Kationenaustauscherharz, das aus Kieselgelpartikeln, quervernetzter Agarose oder quervernetzten Polymethacrylatpolymeren, derivatisiert mit Carboxyl- oder Carboxyalkylgruppen, besteht;
(b) Waschen des Kationenaustauscherharzes zur Entfernung von Nicht-rDSPAα1-Proteinen und Nicht-Protein-Verunreinigungen;
(c) selektives Eluieren des gebundenen rDSPAα1 vom Kationenaustauscherharz mit einem Puffer, der 50 mM Na-phosphat sowie zwischen 100 mM und 500 mM NaCl enthält, oder mit einem Puffer von äquivalenter lonenstärke;
(d) Auftragen des rDSPAα1-haltigen Eluats aus Schritt (c) bei einem pH-Wert zwischen 3 und 5 auf ein Harz mit hydrophober Wechselwirkung, das aus Kieselgelpartikeln, quervernetzter Agarose oder quervernetzten Polymethacrylatpolymeren besteht;
(e) Waschen des Harzes mit hydrophober Wechselwirkung zur Entfernung von Nicht-rDSPAα1-Protein und Nicht-Protein-Verunreinigungen;
(f) selektives Eluieren des gebundenen rDSPAα1 vom Harz mit hydrophober Wechselwirkung unter Verwendung eines Puffers mit 20 mM HCl und mit einer Ethanolkonzentration von mehr als 25%;
(g) Auftragen des rDSPAα1-haltigen Eluats aus Schritt (f) bei einem pH-Wert zwischen 1 und 4 auf ein Affinitätschromatographieharz, das aus einem quervernetzten Polymer aus Allyl-Dextran und N,N'-Methylen-bisacrylamid in Perlenform mit einem Perlendurchmesser zwischen 25 und 75 µm besteht;
(h) Waschen des Affinitätschromatographieharzes zur Entfernung von Nicht-rDSPAα1-Protein und Nicht-Protein-Verunreinigungen;
(i) selektives Eluieren des gebundenen rDSPAα1 vom Affinitätschromatographieharz mit einem Puffer mit 200 mM Glycin bei einem pH-Wert zwischen 3 und 6 oder mit einem Puffer von äquivalenter lonenstärke zur Herstellung von reinem rDSPAα1 in einer wäßrigen Lösung.

23. Verfahren zur Isolierung und Reinigung von rDSPAα1 aus einem biologischen Medium, wobei das Verfahren die folgenden Schritte umfaßt:
(a) Auftragen des Mediums bei einem pH-Wert von 5 auf ein Kationenaustauscherharz, das aus einer Matrix aus Kieselgelpartikeln, die kovalent an Polyethyleniminosilan, worin die Aminogruppen mit Carboxylgruppen derivatisiert sind, gebunden sind, besteht;
(b) Waschen des Kationenaustauscherharzes mit einem pH-Wert von 5 mit 100 mM NaOAc und 50 mM NaPO₄ mit einem pH-Wert von 7,5 zur Entfernung von NichtrDSPAα1-Proteinen und Nicht-Protein-Verunreinigungen;
(c) Eluieren des gebundenen rDSPAα1 vom Kationenaustauscherharz mit einem Puffer, der 50 mM Natriumphosphat sowie 500 mM Natriumchlorid enthält, bei einem pH-Wert von 7,5;
(d) Auftragen des rDSPAα1-haltigen Eluats aus Schritt (c) bei einem pH-Wert von 4 auf ein Harz mit hydrophober Wechselwirkung, das aus halbfesten kugelförmigen Perlen, die mittels Copolymerisation von Ethylenglykol und mit Butylgruppen derivatisierten Polymeren vom Methacrylat-Typ synthetisiert werden, besteht;
(e) Waschen des Harzes mit hydrophober Wechselwirkung mit 20 mM HCl und danach mit 20 mM HCl mit 19% EtOH zur Entfernung von Nicht-rDSPAα1-Protein und Nicht-Protein-Verunreinigungen;
(f) Eluieren des gebundenen rDSPAα1 vom Harz mit hydrophober Wechselwirkung mit einem Puffer, der 29% Ethanol sowie 20 mM Salzsäure enthält, bei einem pH-Wert von 2,5;
(g) Auftragen des rDSPAα1-haltigen Eluats aus Schritt (f) bei einem pH-Wert von 2,5 auf ein Affinitätschromatographieharz, das aus einem quervernetzten Polymer aus Allyl-Dextran und N,N'-Methylen-bisacrylamid, durch das globuläre Proteine zwischen 20 000 und 8 000 000 kDa aufgetrennt werden, besteht;
(h) Waschen des Affinitätschromatographieharzes mit 20 mM HCl zur Entfernung von Nicht-rDSPAα1-Protein und Nicht-Protein-Verunreinigungen;
(i) Eluieren des gebundenen rDSPAα1 vom Affinitätschromatographieharz mit einem Puffer, der 200 mM Glycin enthält, bei einem pH-Wert zwischen 4 und 5 zur Herstellung von reinem rDSPAα1 in einer wäßrigen Lösung.

## Revendications

1. Procédé de purification de l'activateur du plasminogène salivaire Desmodus rotundus recombinant (rDSPA α1) à partir d'un milieu biologique, le procédé comprenant les étapes suivantes :
(a) l'application du milieu sur une résine échangeuse de cations à un pH compris entre 4 et 7 ;
(b) le lavage de la résine échangeuse de cation en vue d'éliminer les protéines non-rDSPA α1 et les contaminants non protéiques ;
(c) l'élution sélective du rDSPA α1 lié à partir de la résine échangeuse de cation ;
(d) l'application de l'éluant contenant le rDSPA α1 de l'étape (c) sur une résine à interaction hydrophobe à un pH compris entre 3 et 5 ;
(e) le lavage de la résine à interaction hydrophobe en vue d'éliminer les protéines non-rDSPA α1 et les contaminants non protéiques ;
(f) l'élution sélective du rDSPA α1 lié à partir de la résine à interaction hydrophobe ;
(g) l'application de l'éluant contenant le rDSPA α1 de l'étape (f) sur une résine de chromatographie d'affinité à un faible pH et une faible force ionique ;
(h) le lavage de la résine de chromatographie d'affinité en vue d'éliminer les protéines non rDSPA α1 et les contaminants non protéiques ;
(i) l'élution sélective du rDSPA α1 lié à partir de la résine de chromatographie d'affinité en vue de produire le rDSPA α1 pur en solution aqueuse.

2. Procédé selon la revendication 1, dans lequel le milieu biologique est un milieu conditionné.

3. Procédé selon la revendication 1, dans lequel la résine échangeuse de cations dans l'étape (a) est constituée de particules de gel de silice, d'agarose réticulé ou de polymères polyméthacrylates réticulés, dérivatisés avec des groupes carboxy ou carboxyalkyle.

4. Procédé selon la revendication 3, dans lequel la résine échangeuse de cations est constituée d'une matrice de particules de silice liées par covalence à un polyéthylène-iminesilane, dans lequel les groupes amino du polyéthylène-iminesilane ont été dérivatisés avec des groupes carboxy.

5. Procédé selon la revendication 3, dans lequel les conditions de charge dans l'étape (a) comprennent l'application des milieux à un pH compris entre 4 et 7.

6. Procédé selon la revendication 4, dans lequel l'élution du rDSPA α1 dans l'étape (c) est réalisée en utilisant un tampon contenant 50 mM de NaPhos et entre 100 mM et 500 mM de NaCl, ou un tampon de force ionique équivalente.

7. Procédé selon la revendication 1, dans lequel la résine à interaction hydrophobe dans l'étape (d) est une résine non chargée, dérivatisée avec des chaînes alkyle d'une longueur de 1 à 10 carbones ou avec des groupes aryl-alkyle.

8. Procédé selon la revendication 7, dans lequel la résine non chargée est constituée de particules de gel de silice, d'agarose réticulée ou d'un polymère polyméthacrylate réticulé.

9. Procédé selon la revendication 7, dans lequel la résine non chargée est constituée de billes sphériques semi-rigides synthétisées par une copolymérisation de l'éthylèneglycol et de polymères du type méthacrylate, dérivatisés avec des groupes butyle.

10. Procédé selon la revendication 9, dans lequel les conditions de charge dans l'étape (d) comprennent l'application de l'éluant de l'étape (c) à un pH compris entre 3 et 5.

11. Procédé selon la revendication 9, dans lequel les conditions de charge dans l'étape (d) comprennent l'application de l'éluant de l'étape (c) dans 50 mM de NaPhos, 500 mM de NaCl, à pH ajusté à 4 avec de l'acide phosphorique, ou dans un tampon de force ionique équivalente.

12. Procédé selon la revendication 9, dans lequel l'élution dans l'étape (f) est réalisée en utilisant un tampon contenant 20 mM de HCl et présentant une concentration en éthanol supérieur à 25 %.

13. Procédé selon la revendication 12, dans lequel la concentration en éthanol est comprise entre 28,5 % et 30 %.

14. Procédé selon la revendication 12, dans lequel la concentration en éthanol est de 29 %.

15. Procédé selon la revendication 1, dans lequel la résine de chromatographie d'affinité de l'étape (g) est constituée d'un copolymère réticulé d'allyldextrane et de N,N'-méthylène-bis-acrylamide sous la forme de billes présentant un diamètre compris entre 25 et 75 µm.

16. Procédé selon la revendication 15, dans lequel les billes sont capables de fractionner des protéines globulaires de 20 000 à 8 000 000 kDa.

17. Procédé selon la revendication 15, dans lequel les conditions de charge dans l'étape (g) comprennent l'application de l'éluant de l'étape (f) à un pH compris entre 1 et 4.

18. Procédé selon la revendication 15, dans lequel l'élution dans l'étape (i) est réalisée en utilisant un tampon contenant 200 mM de glycine à un pH compris entre 3 et 6, ou un tampon de force ionique équivalente.

19. Procédé selon la revendication 1, comprenant en outre la concentration de la solution aqueuse de rDSPA α1.

20. Procédé selon la revendication 19, comprenant en outre la lyophilisation de la solution de rDSPA α1 concentrée.

21. Procédé selon la revendication 1, comprenant en outre la concentration de la solution aqueuse de rDSPA α1 et la lyophilisation de la solution de rDSPA α1 concentrée.

22. Procédé selon la revendication 1, le procédé comprenant les étapes suivantes :
(a) l'application du milieu à un pH compris entre 4 et 7, sur une résine échangeuse de cations constituée de particules de gel de silice, d'agarose réticulé, ou de polymères polyméthacrylates réticulés, dérivatisés avec des groupes carboxy ou carboxyalkyle ;
(b) le lavage de la résine échangeuse de cation en vue d'éliminer les protéines non-rDSPA α1 et les contaminants non protéiques ;
(c) l'élution sélective du rDSPA α1 lié à partir de la résine échangeuse de cation en utilisant un tampon contenant 50 mM de phosphate de sodium et entre 100 mM et 500 mM de NaCl, ou un tampon de force ionique équivalente ;
(d) l'application de l'éluant contenant le rDSPA α1 de l'étape (c) à un pH compris entre 3 et 5 sur une résine à interaction hydrophobe constituée de particules de gel de silice, d'agarose réticulé ou de polymères polyméthacrylates réticulés ;
(e) le lavage de la résine à interaction hydrophobe en vue d'éliminer les protéines non-rDSPA α1 et les contaminants non protéiques ;
(f) l'élution sélective du rDSPA α1 lié à partir de la résine à interaction hydrophobe en utilisant un tampon contenant 20 mM de HCl et présentant une concentration en éthanol supérieure à 25 % ;
(g) l'application de l'éluant contenant le rDSPA α1 de l'étape (f) à un pH compris entre 1 et 4 sur une résine de chromatographie d'affinité constituée d'un polymère réticulé d'allyldextrane et de N,N'-méthylènebis-acrylamide sous la forme de billes présentant un diamètre compris entre 25 et 75 µM ;
(h) le lavage de la résine de chromatographie d'affinité en vue d'éliminer les protéines non rDSPA α1 et les contaminants non protéiques ;
(i) l'élution sélective du rDSPA α1 lié à partir de la résine de chromatographie d'affinité avec un tampon contenant 200 mM de glycine à un pH compris entre 3 et 6 ou un tampon de force ionique équivalente, en vue de produire le rDSPA α1 pur en solution aqueuse.

23. Procédé d'isolation et de purification du rDSPA α1 à partir d'un milieu biologique, le procédé comprenant les étapes suivantes :
(a) l'application du milieu à un pH de 5 sur une résine échangeuse de cations constituée d'une matrice de particules de silice liées par covalence à un polyéthylène-iminesilane, dans lequel les groupes amino ont été dérivatisés avec des groupes carboxy ;
(b) le lavage de la résine échangeuse de cations à un pH de 5 avec du NaOAC 100 mM et du NaPO₄ 50 mM à un pH de 7,5, en vue d'éliminer les protéines non-rDSPA α1 et les contaminants non protéiques ;
(c) l'élution du rDSPA α1 lié à partir de la résine échangeuse de cations avec un tampon contenant 50 mM de phosphate de sodium et 500 mM de chlorure de sodium à un pH de 7,5 ;
(d) l'application de l'éluant contenant le rDSPA α1 de l'étape (c) à un pH de 4 sur une résine à interaction hydrophobe constituée de billes sphériques semi-rigides synthétisées par une copolymérisation de l'éthylèneglycol et de polymères du type méthacrylate dérivatisés avec des groupes butyle ;
(e) laver la résine à interaction hydrophobe avec du HCl 20 mM et ensuite avec du HCl 20 mM contenant 19 % de EtOH en vue d'éliminer les protéines non-rDSPA α1 et les contaminants non protéiques ;
(f) l'élution du rDSPA α1 lié à partir de la résine à interaction hydrophobe avec un tampon contenant 29°% d'éthanol et 20 mM d'acide chlorhydrique à un pH de 2,5 ;
(g) l'application de l'éluant contenant le rDSPA α1 de l'étape (f) à un pH de 2,5 sur une résine de chromatographie d'affinité constituée d'un polymère réticulé d'allyldextrane et de N,N'-méthylène-bis-acrylamide qui fractionne des protéines globulaires de 20 000 à 8 000 000 kDa ;
(h) le lavage de la résine de chromatographie d'affinité avec du HCl 20 mM en vue d'éliminer les protéines non-rDSPA α1 et les contaminants non protéiques ;
(i) l'élution du rDSPA α1 lié à partir de la résine de chromatographie d'affinité avec un tampon contenant 200 mM de glycine à un pH compris entre 4 et 5, en vue de produire le rDSPA α1 pur en solution aqueuse.
